# EUROPEAN PATENT SPECIFICATION

(11) **EP 1 691 733 B1**
(45) Date of publication and mention of the grant of the patent: **23.04.2008**
(21) Application number: 04798816.7
(22) Date of filing: 10.11.2004
(51) Int. Cl.: A61F 2/46

(54) **INSERTER FOR MINIMALLY INVASIVE JOINT SURGERY**
EINFÜHRELEMENT FÜR DIE MINIMALINVASIVE GELENKCHIRURGIE
PORTE-PROTHESE POUR CHIRURGIE ARTICULAIRE TRES PEU INVASIVE

(30) Priority: 10.11.2003 US 518768 P; 26.02.2004 US 548542 P; 10.04.2004 US 561141 P
(43) Date of publication of application: 23.08.2006
(73) Proprietor: PRECIMED S.A., 2534 Orvin (CH)
(72) Inventor: LECHOT, André, CH-2534 Orvin (CH); DESARZENS, Yves, CH-2606 Corgémont (CH)
(74) Representative: Mötteli-Mantelli, Novella
(86) International application number: PCT/IB2004/003676
(87) International publication number: WO 2005/044153

(56) References cited:
- WO-A-01/06964
- US-A- 4 305 394
- US-A- 5 584 837

## Description

### Background of the Invention

This invention relates to surgical inserters for aiding in installing orthopedic prostheses, and, more particularly, to easily sterilizable inserters for installing acetabular implants in the acetabular socket, to which the closest prior art is WO 0106964 A.

Complicated mechanical devices have crevices and recesses that are difficult, if not almost impossible to clean with ease. Devices that are not properly cleaned and sterilized run the risk of disease transfer from patient to patient following the emergence of certain "prions" that are not killed by normal hospital sterilisation and need to be physically removed by washing / rinsing.

Further, in surgical procedures in which access to the treatment site is limited, it is difficult to use current solutions without subjecting the patient to repeated abrasion and tissue trauma when inserting and extracting surgical instruments.

Further, the insertion of the implant is often problematic, and the orientation of the implant, particularly any fixing holes that might be pre-drilled in the implant is often critical to minimize recovery time of the patient. Still further, once the appropriate position of the implant is selected, it is often difficult to ensure that the position does not change upon insertion of the assembly through the incision.

What is needed therefore is an inserter that is easily adjustable, disassemblable, and cleanable. Further, what is needed is an inserter that enables the surgeon to better maneuver position and install an implant in a particular angular orientation.

### Summary of the Invention

An acetabular impactor according to the present invention is defined in claim 1.

An acetabular inserter aids a surgeon in controlling the installation of an acetabular cup prosthesis generally having a central, female aperture. The inserter has a housing which encloses a drive train having, at a far end, a prosthesis engaging interface (e.g., a thread), and at the opposite end, a handle which facilitates turning of the drive train by the operator.

The inserter enables easy orientation of a prosthesis attached to its end, which is important because the prosthesis often has pre-drilled holes and thus, these must be properly positioned prior to fastening through these holes.

An objective of the invention is to be "easily cleaned" by quick and modular disassembly which enables access to all surfaces that they can be cleaned, the reduction in number of small radius internal corners, crevices and small gaps and the absence of blind holes.

Another object of the invention is to provide an inserter which enables the implant to be locked in an angular orientation prior to installation of the implant.

Another object of the invention is to provide a dual mechanism that uses common components to lock the implant in place as well as to provide for easy disassembly for cleaning and sterilization.

Another object of the invention is to minimise the number of pieces and the risk that parts could be lost.

### Brief Description of the Drawings

The attached drawings represent, by way of example, different embodiments of the subject of the invention.
FIG. **1A** is a cross-sectional side view of the inserter of the invention.
FIG. **1B** is a side view of the inserter of the invention.
FIG. **1C** is a perpective view of the inserter of the invention showing a one way catch mechanism.
FIG. **2A** is an operational side view of the inserter of the invention.
FIG. **2B** is an operational back view of the inserter of the invention.
FIG. **3A** is a perspective view of the inserter of the invention, showing a step of disassembly for cleaning.
FIG. **3B** is a perspective view of the inserter of the invention, showing another step of disassembly for cleaning.
FIG. **3C** is a perspective view of the inserter of the invention, showing a stage of disassembly for cleaning.
FIG. **3D** is a perspective view of the inserter of the invention, showing a stage of reassembly after cleaning.
FIG. **4** is a schematic view of a prior art inserter.
FIG. **5** is a schematic view of the inserter of the invention in operation.
FIG. **6A** is a side, cross sectional view of an alternate embodiment of the inserter of the invention.
FIG. **6B** is a perspective view of the jaws of the inserter of the invention.
FIG. **7A** is a side, cross sectional view of the tip of the inserter of the invention.
FIG. **7B** is a perspective cross sectional view of the tip of the inserter of the invention.
FIG. **8** is a perspective view of an actuation screw of the inserter of the invention.
FIG. **9A** is a cross-sectional side view of another alternate embodiment of the inserter of the invention, taken along line **A-A** in FIG. **9B****.**
FIG. **9B** is top view of the invention.
FIG. **10A** is a perspective view of the invention.
FIG. **10B** is a closeup perspective view of the invention, showing the dual locking mechanism.
FIG. **10C** is a section view of area 10C in FIG. **9A****.**

### Detailed Description of the Preferred Embodiment(s)

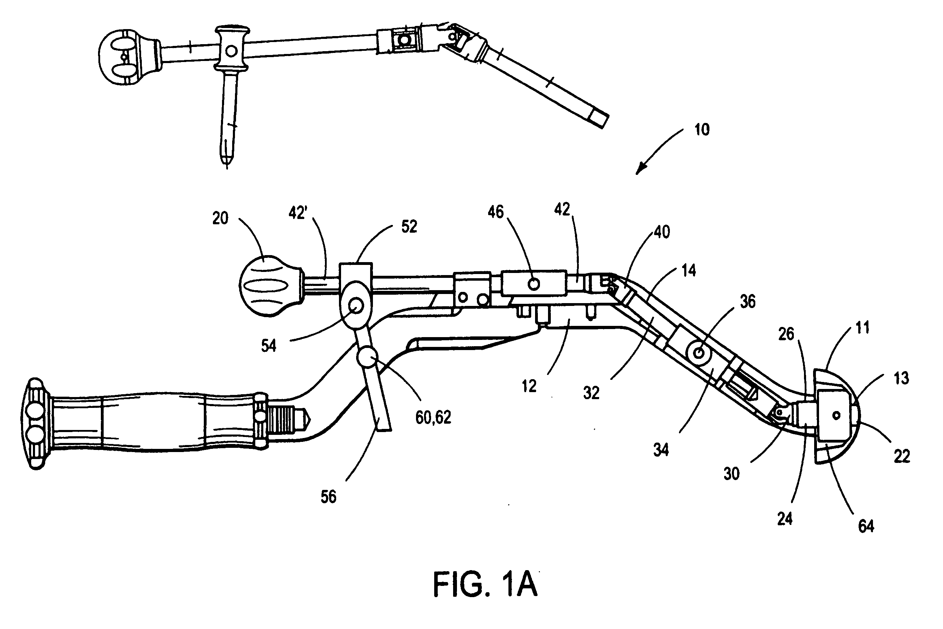

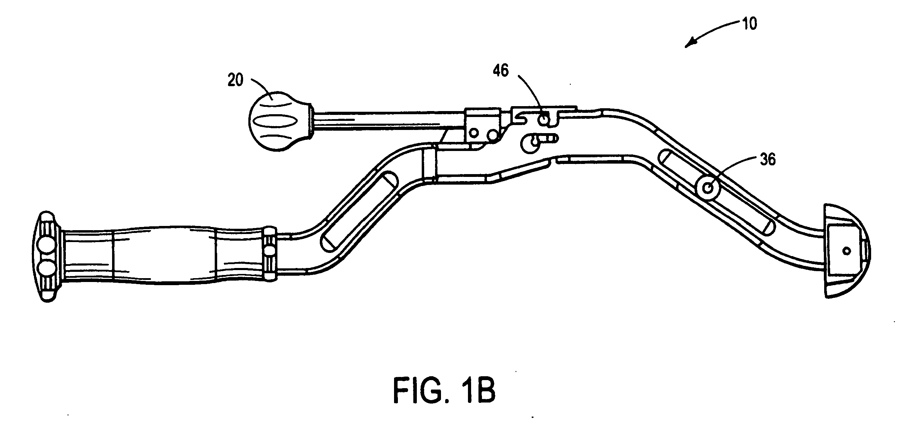

Referring now to FIGs. **1A-1C**, an acetabular inserter 10 is provided to aid the surgeon in controlling the installation of an acetabular cup prosthesis 11 having a central, female aperture 13. The inserter 10 has a housing 12 which encloses a drive train 14 having, at a far end, a prosthesis engaging interface 16 (preferably threaded), and at the opposite end, a handle 20 which facilitates turning of the drive train by the operator. The housing 12 may be C-shaped, as shown, in order to minimize the invasiveness of the surgery by better clearing anatomical structures and tissue.

The interface 16 is cut on a boss 22 on a cylindrical piston 24 which slides in an axial hole 26 in the housing 12. The interface 16 is preferably threaded. The piston 24 is connected by way of a first U-joint 30 to a lever 32 which slides in a pivoting sleeve 34 fixed to the housing 12 via a pivot 36. The lever 32 is connected via a second U-joint 40 to a second pivoting lever 42 which is fixed to pivot in a catch 44 on a pivot pin 46. The catch 44 is essentially a divot or a seat cut into the housing 12, against which the pivot pin 46 of the lever 42 is captured when a slide 50 is slide over the pin when engaged against the seat.

A slideable sleeve 52 slides over the lever 42 and has a trunion 54 to which a rod 56 is pivotally attached. The rod 56 passes through a one-way catch 60 in the housing 12. The one-way catch 60 can be a captured split wedge sleeve 62 having an inner diameter that just matches the outer diameter of the rod 56 and which is captured in a recess having a matching conical surface that surrounds the sleeve so as to allow the rod 56 to slide into the housing 12, but to prevent the rod from sliding out of the housing unless an unlock lever (not shown) is activated, such lever merely lifting the sleeve 62 out of engagement with the conical surface so as not to lock and to permit the rod to back out of the housing. Any number of alternative one way lock devices may be used however, the selection of which being within the skill of a person of ordinary skill in this field.

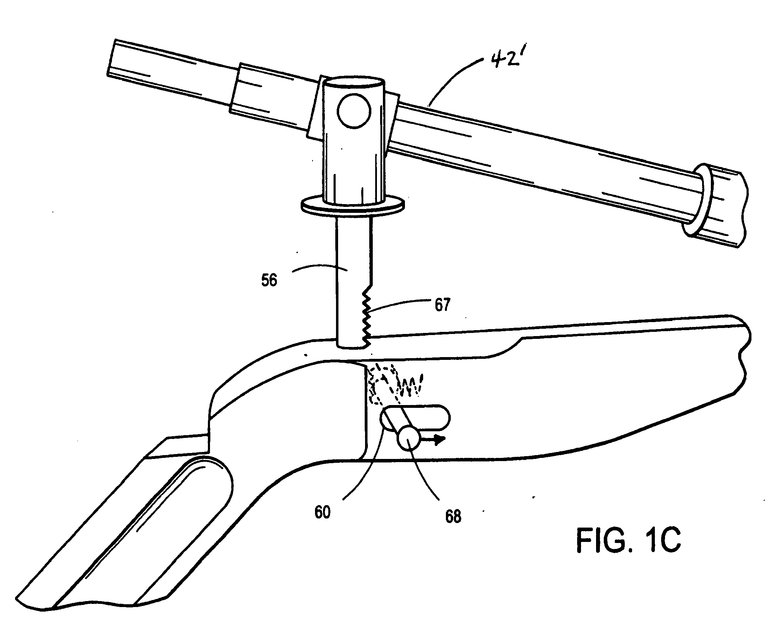

Referring now to FIG. **1C****,** an alternative embodiment of the one way catch mechanism 60 is shown. In this embodiment, the rod 56 passes through a one-way catch 60 in the housing 12. The one-way catch 60 has an inner recess that matches the outer diameter of the rod 56. The inner recess has a ratchet pawl (not shown) that locks against one way ratchet teeth 67 so as to allow the rod 56 to slide into the housing 12, but to prevent the rod from sliding out of the housing unless an unlock lever 68 is activated, such lever merely pulling the pawl away from the teeth to permit the rod to back out of the housing.

A polymeric impactor head 64 is molded over the end of the housing 12, to absorb the impact stresses incurred during use as an impactor. The head 64 is selected so as to have good frictional characteristics as well. Nevertheless, a metal, non-molded head may also be used with satisfactory results.

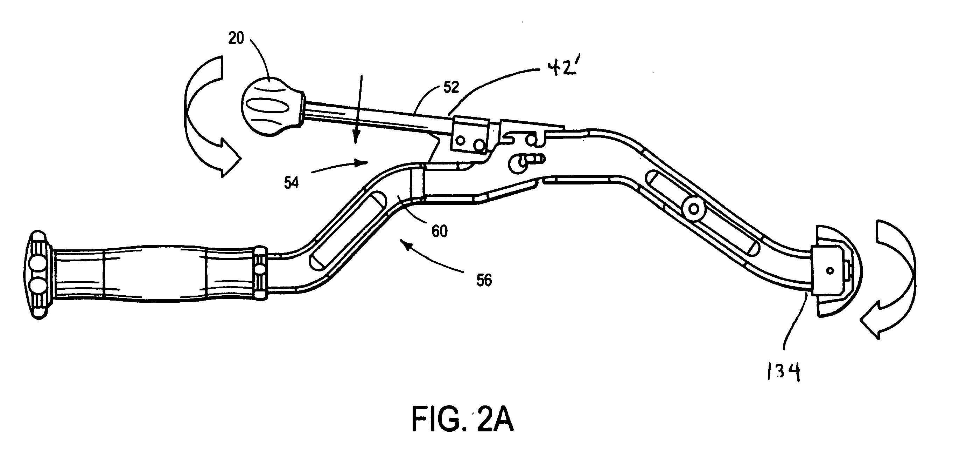

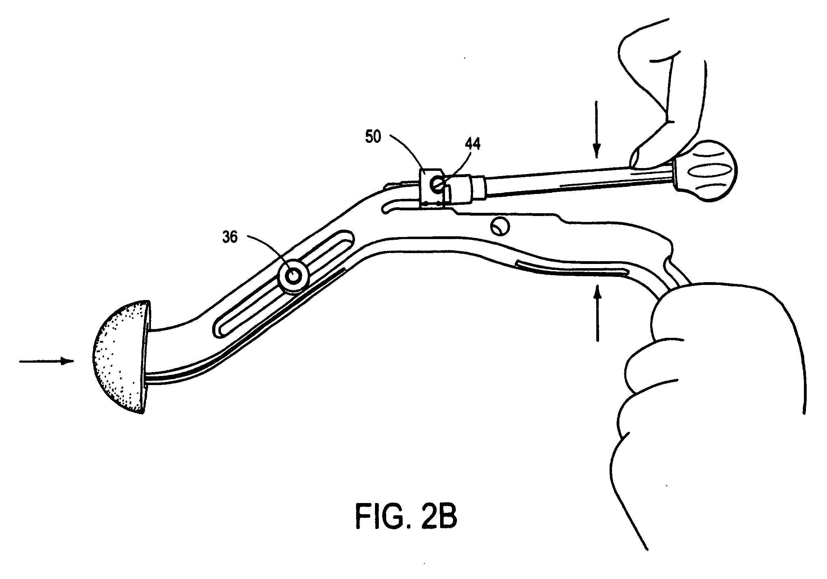

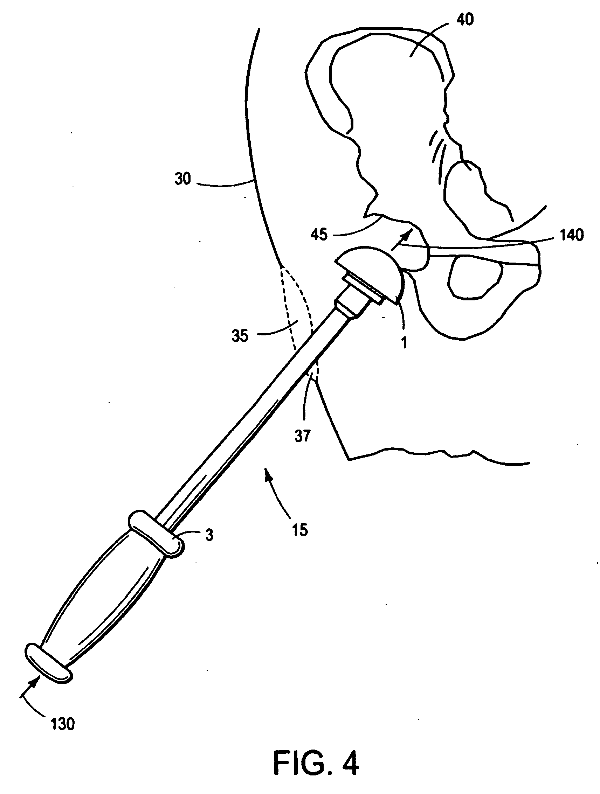

Referring now to FIGS. **2A - 2B****,** in operation, the interface 16 (preferably threaded) of the piston 24 is engaged with the hole 13 of the prosthesis 11. The operator may rotate the handle 20 about its axis to turn the drive train 14 in order to interface the piston 24 into the hole 13 or to orient the prosthesis in what he believes to be a correct or an initial position. Then, an end 42' of the lever 42 is urged downwardly toward the housing 12. Such downward movement acts through the drive train 14 to draw the piston 24 into the housing 12, and thus to cause the inner surface of the prosthesis 11 to be drawn against the head 64 so as to create a normal force between the inside of the prosthesis and the head so as to prevent rotation of the prosthesis 11 relative to the housing 12. The operator may use the one way locking mechanism 62 to lock the lever 42 in a position so as to lock the prosthesis 11 against the head 64, thus enabling the surgeon to pre-set and lock the position of the prosthesis prior to the installation thereof. Note that orientation of the prosthesis 11 is important because the prosthesis often has pre-drilled holes 4 (shown in FIG. **4A**) and thus, these must be properly positioned prior to fastening through these holes.

The "easily cleaned" objective of the invention enables access to all surfaces that they can be cleaned (parts covering another part can be moved or removed to expose all surfaces), the reduction in number of small radius internal corners, crevices and small gaps and the absence of blind holes.

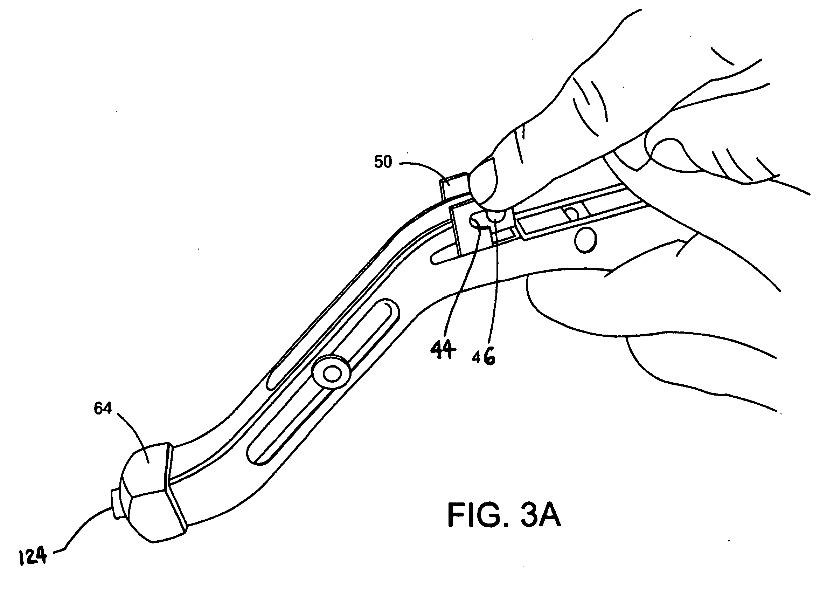

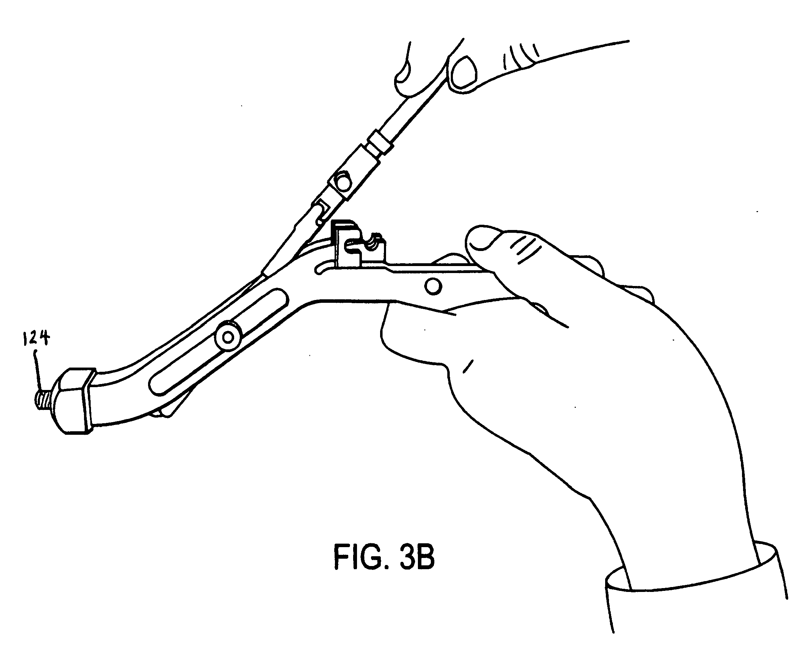

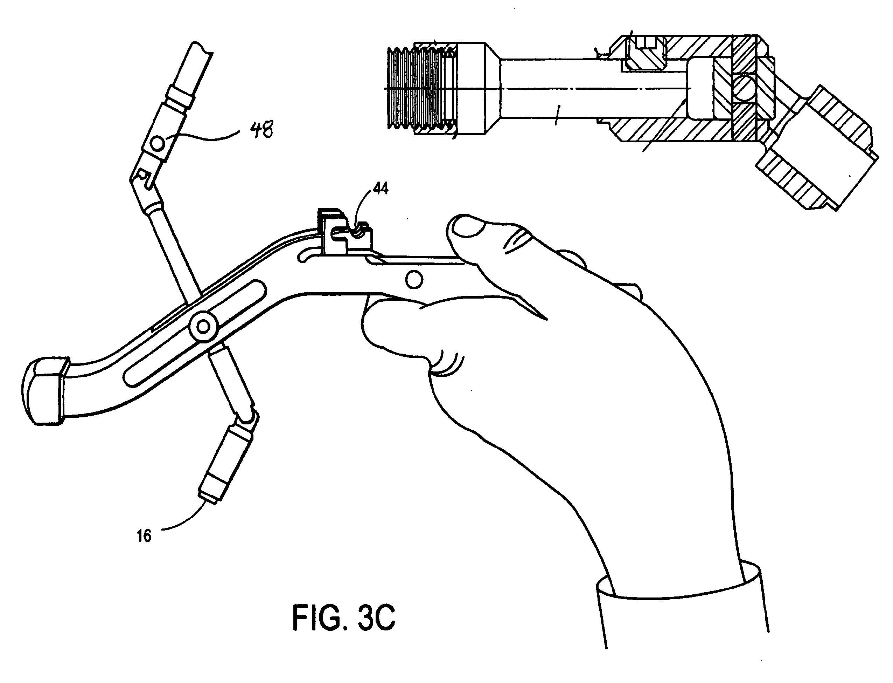

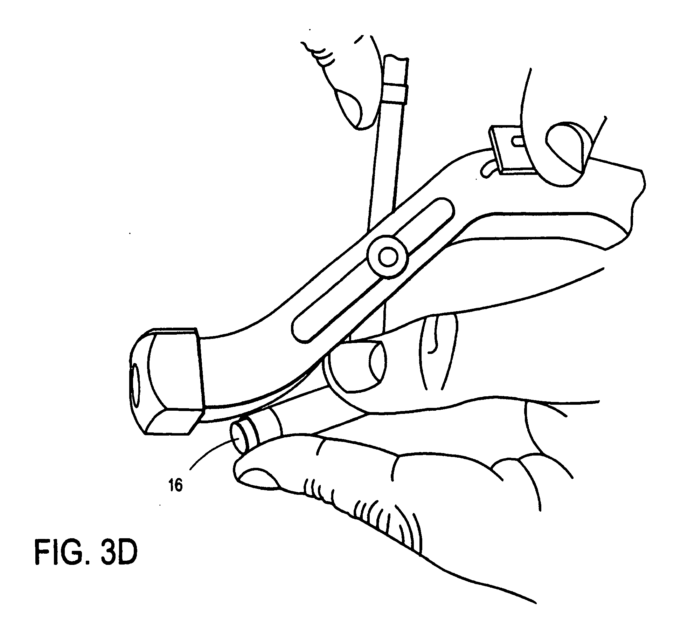

Referring now to FIGS. **3A - 3D****,** in the embodiment shown, the device 10 is disassembled for cleaning by simply sliding the slide 50 back so as to release the pivot 46 and then lift the drive train 14 out of the housing but allow it to remain pivotally connected at pivot 36. As the drive train 14 is pivoted, the piston 16 is drawn out of the hole 26 in the housing 12. To reassemble after cleaning, the piston 16 is reinserted into the hole 26 and the drive train 14 is rotated back into position, with the one way locking mechanism entering its receiver and the pivot 46 again entering into the catch 44. The slide 50 is then slide over the pivot 46 and the inserter 10 is again ready for use.

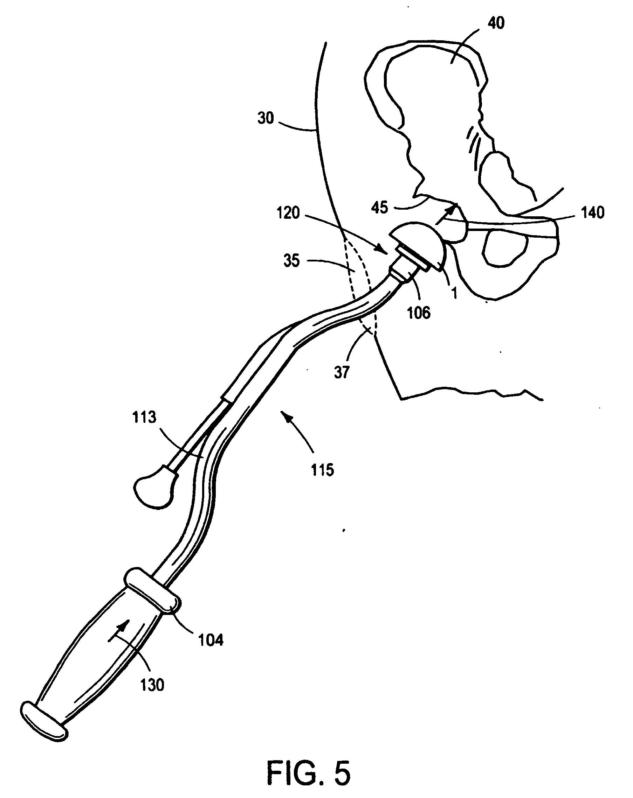

Referring to FIGS. **4-5****,** the inserter 115 and a prior art inserter 15, respectively, are shown passing through a miniature incision 35 in the patient's skin 30. In FIG. **4**, the inserter 15 is shown approaching the acetabulum 40 in an orientation desirable to ream the socket 45. The difficulty with the prior art spindle 15 is shown as the shaft 3 impinges on the miniature incision 35 at edge of the incision 37. The current surgical protocols are being pushed to the limits and the incision sizes are being reduced in the hopes of increasing the patient's speed to recovery. In some cases surgeons are using a two-incision approach, one to reach the acetabulum and the other to reach the femur. Either one incision or two incision techniques demand less trauma to the patient requiring the instruments to be more optimally designed to make up for the lack of operating space. The reamer of FIG. **5** shows a new inserter 115, which has a bent housing 113 containing the drive shaft 107.

It is important to place the bends in the housing at critical locations to pass through the miniature incision without impinging on the skin 30 at 37 while still maintaining the same surgical protocol. The reason why the drive end 104 and the holding mechanism 120 need to be in line or on parallel axis is so that the applied force 130 results in an axial motion 140. This allows the surgeon to maintain the existing technique since inherently inserter 15 in FIG. **4** would give the same result since it has a straight drive shaft 3. This allows the surgeon to apply a load directly along the path of reaming.

It should be noted that a second head (not shown) can be mounted onto the front of the device 10, the head formed so as to conform with a surface of an acetabular cup liner, in order to enable the device to seat a liner as well as the cup.

The attached drawings represent, by way of example, different embodiments of the subject of the invention.

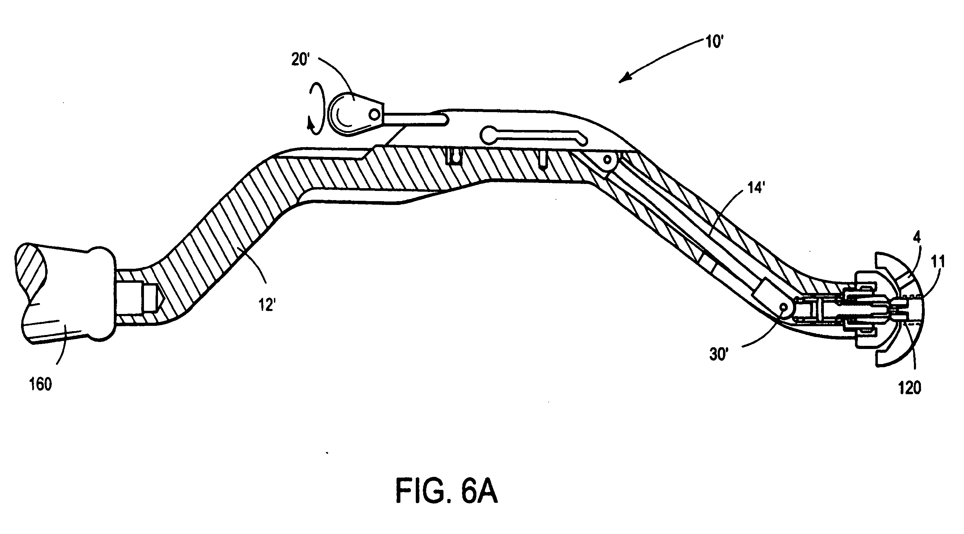

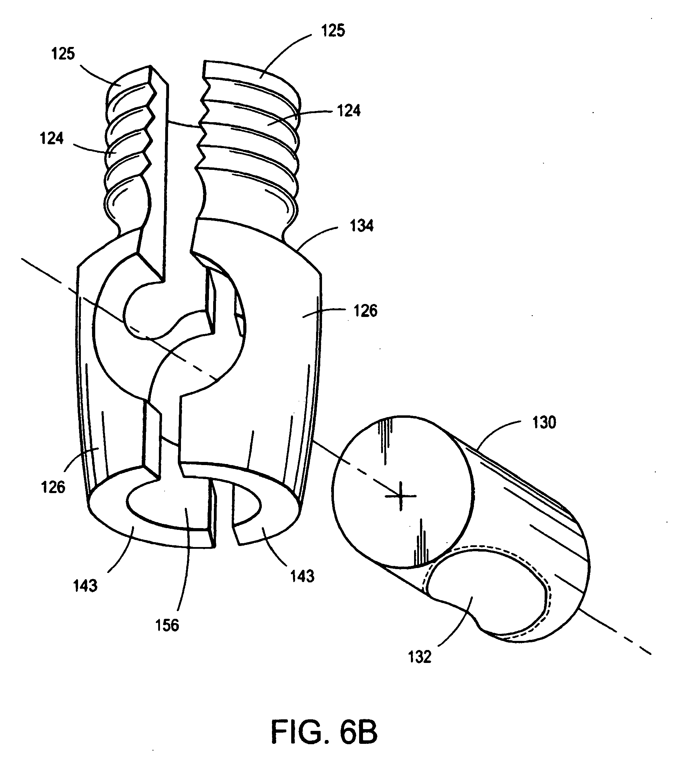

Referring now to FIG. **6A****,** in another embodiment, an acetabular inserter 10' is provided to aid the surgeon in controlling the installation of a hip prosthesis 11. The inserter 10' has a housing 12' which encloses a drive train 14' having, at a far end, a prosthesis engaging collet 120 (shown in detail in FIG. **6B**), and at the opposite end, a knob or handle 20' which facilitates turning of the drive train by the operator. The housing 12' may be C-shaped, as shown, in order to minimize the invasiveness of the surgery by better clearing anatomical structures and tissue.

When the knob 20' is turned in one direction, the prosthesis-engaging collet 120 locks the prosthesis 11 against rotational movement. This enables the surgeon to pre-set and lock the position of the prosthesis 11 prior to the installation thereof. Such selective locking of the prosthesis 11 is important because the prosthesis 11 often has pre-drilled holes 4 and thus, these must be properly positioned prior to fastening through these holes. Further, the collet action of the collet 120 eliminates the need of threading the acetabular prosthesis 11 onto the end of the inserter 10' as the prosthesis can simply be placed over the collet and the collet expanded so as to grip internal threads 122 of the prosthesis 11. Note that to improve likelihood of alignment, the threads 124 on the jaws 126 may be replaced with longitudinally aligned dimples (not shown) having a profile that resembles threads but yet which minimizes the need for precise orientation of the internal threads of the prosthesis and the dimples on the jaws 126 of the collet 120.

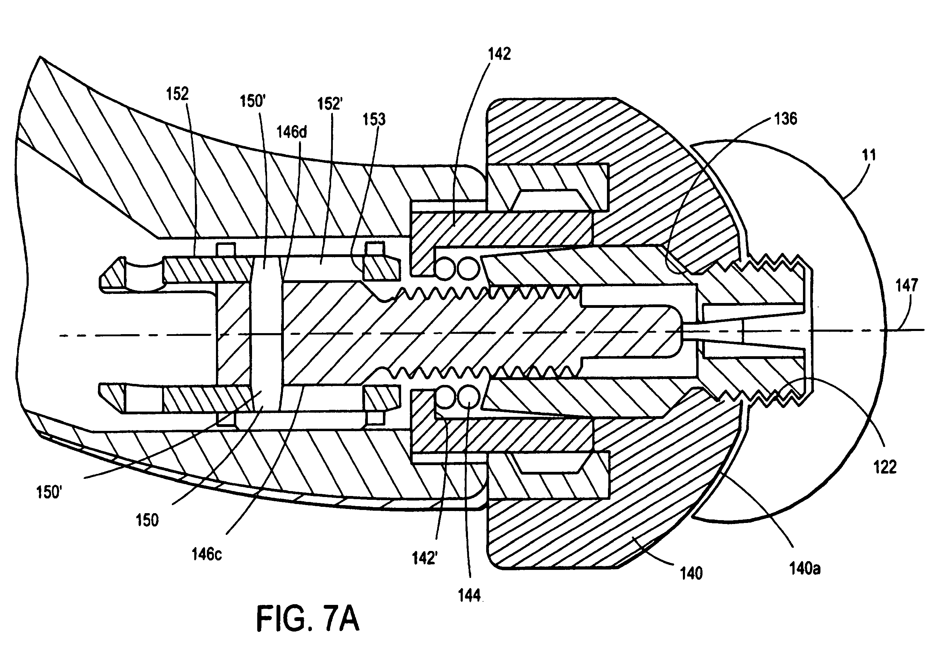

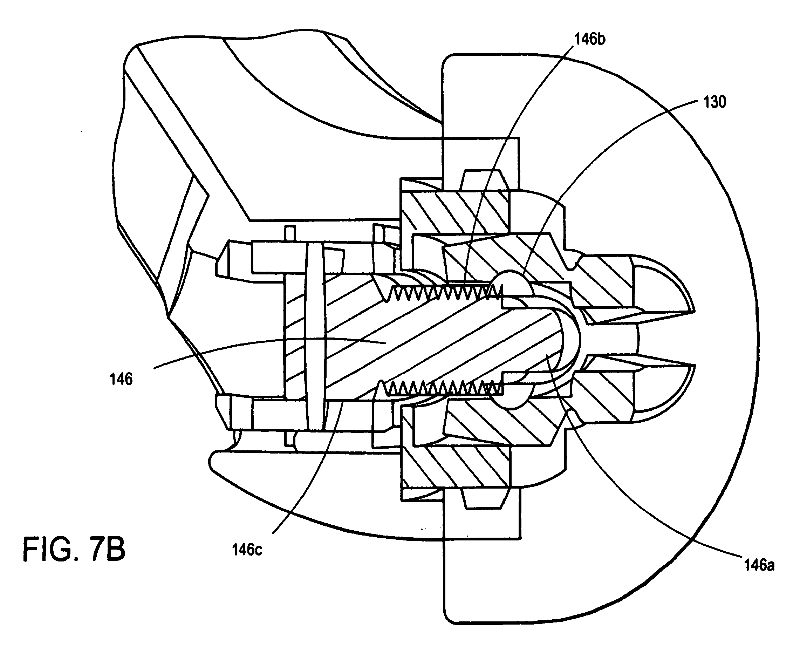

Referring now to FIGs. **6B****,** **7A**, and **7B****,** the prosthesis-engaging collet 120 is made up of two jaws 126 which pivot on a ball or cylindrical pivot 130 having an threaded bore 34 passing transversely therethrough. The jaws 126 are constrained at one end 30' by a shoulder 136 of a plastic impaction head 140, along their outer circumference by a cup-shaped sleeve 142, and at an opposite end 143 by a compression spring 144 which reacts against a bottom end 142' of the sleeve 142.

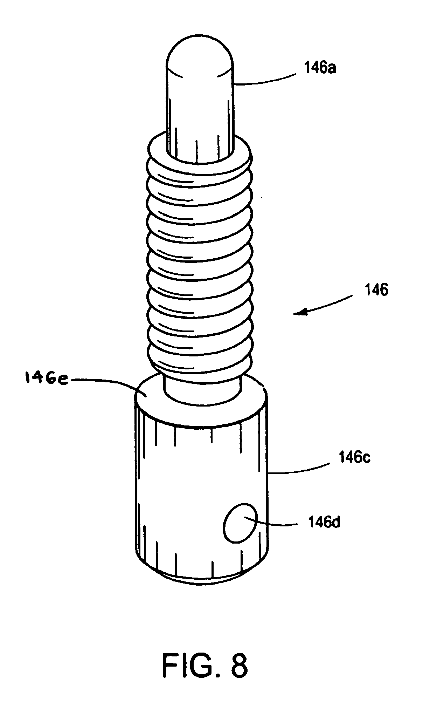

Referring as well to FIG. **8**, an actuation screw 146 has a narrow cylindrical tip 146a, a threaded body **146b** and a cylindrical interface 146c. The interface 146c has a cross hole 146d formed transversely to the longitudinal axis 147 of the screw 146. A U-joint shackle or clevis 152 slides over the interface 146c. A cross pin 150 passing through slots 152' in the shackle 152 and through the cross hole 146d of the interface 146c so that ends 150' of the cross pin protrude beyond the outermost cylindrical surface of the interface into the slots 152'.

The shackle 152 is part of a u-joint 30' of the drive train 14' which is connected to the knob 20' at an opposite end thereof. When the knob 20' is turned by the operator, the drive train 14' causes the actuation screw 146 to progress forward, and thus the tip 146a is able to move into a space 156 between the jaws 126 so as to progressively open the jaws until they are fully locked open. If the drive train 14' is turned further, then the ends 150' of the cross pin 150 contact an end 153 of the slot 152'at which point further turning causes the collet 120 to draw inward, pulling the prosthesis 11 into snug contact with the face of the impaction head 140. When fully open, the space 156 formed between the jaws 126 just receives the tip 146a and thus has substantially cylindrical boundaries. The external surface of the prosthesis-engaging end of the jaws 126 may take any suitable form so as to help lock the prosthesis 11 in place. A simple form which is effective may be a simple thread-matching profile which could include a pitch or not, depending on whether it is desired that the prosthesis be able to be removed by unthreading, despite the collet 120 being in a substantially locked position.

In operation, the prosthesis first is placed over or threaded onto the collet 120 via a threaded hole 122. In a second step, the prosthesis 11 is oriented with respect to the form of the inserter 10', in order to minimally impact soft tissue. In a third step, the handle 160 of the inserter 10' is gripped and the prosthesis 11 placed through the incision 35. In a fourth step, the inserter 10' is used to impact the prosthesis 11 in place by impacting a rear portion of the inserter with a mallet, for example. Optionally, with the current design, it is envisioned that the prosthesis 11 be inserted into the incision 35 as a first step, potentially taking advantage of being able to more freely maneuver it into the incision and roughly position it prior to inserting the collet 120 of the inserter 10' into a mating hole-this optional procedure is used, the knob 20' of the inserter may then be turned by the operator to actuate the opening of the collet 120 and thus the fixing on the end of the inserter 10'. These optional steps substitute for the above mentioned four steps. In a fifth step, the knob 20' is turned in an opposite direction in order to release the prosthesis 11. In a final step, the inserter 10' is removed from the incision 35.

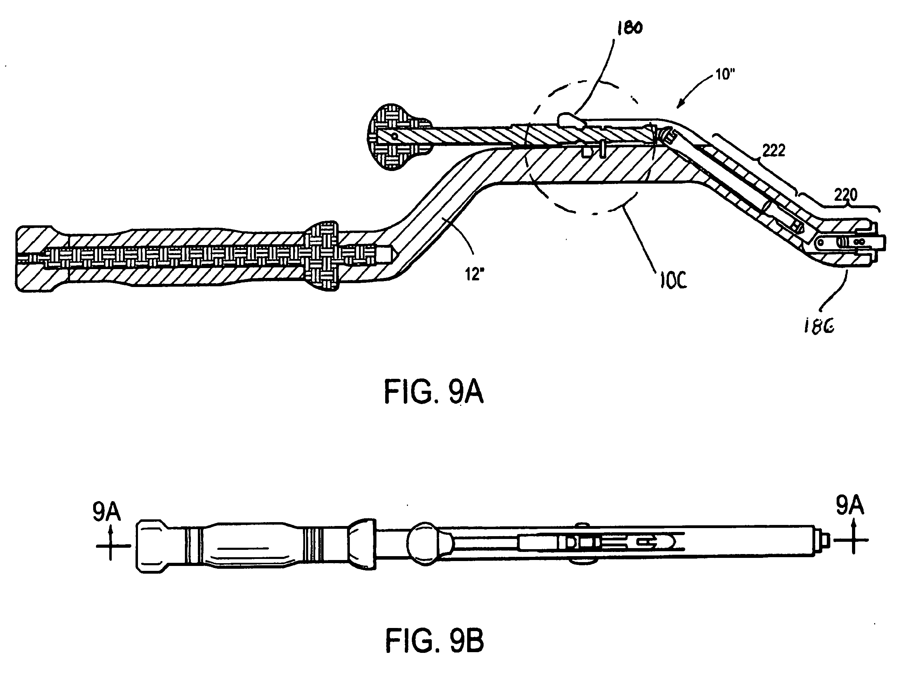

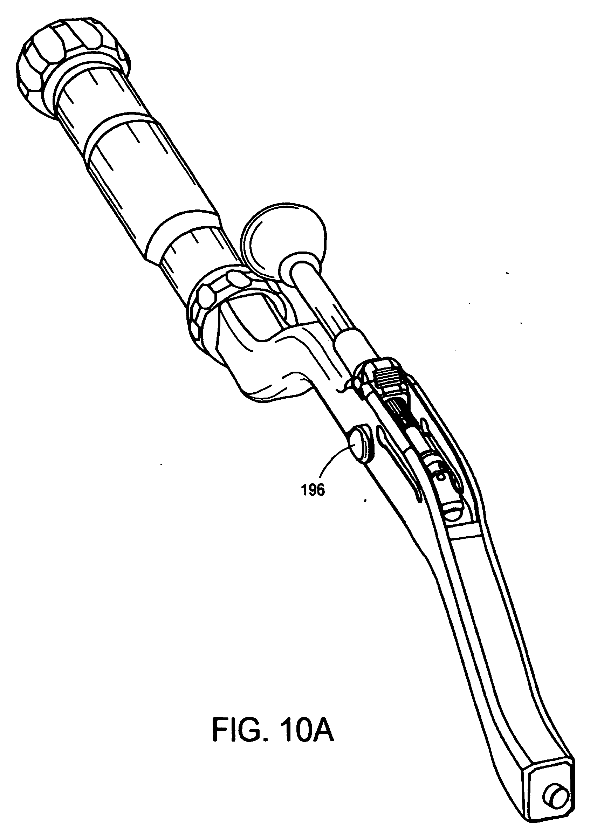

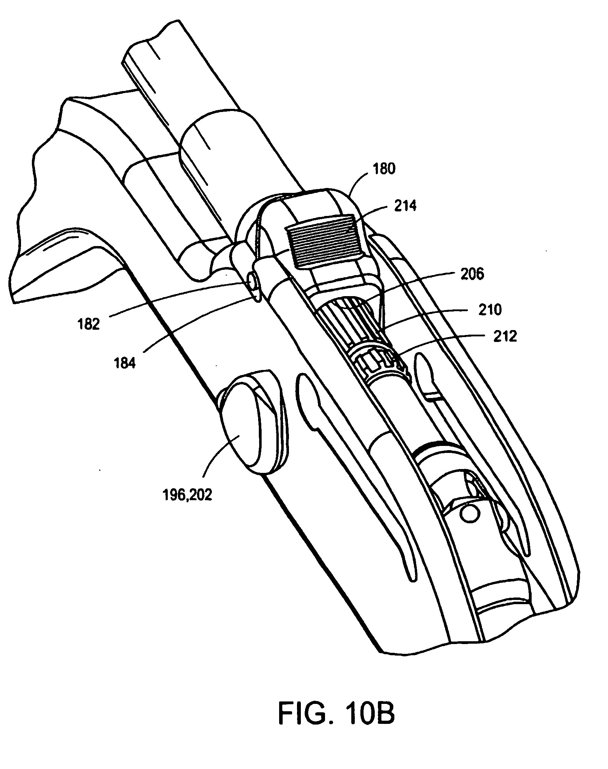

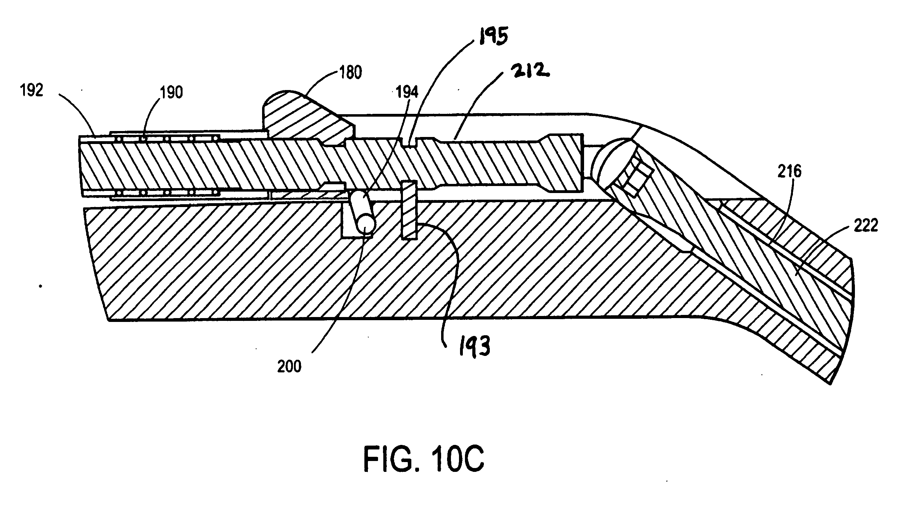

Referring now to FIGs. **9A - 9B****,** and **10A - 10C,** another embodiment of the acetabular inserter 10' is provided to aid the surgeon in controlling the installation of a hip prosthesis 11. The inserter 10" has a housing 12" which encloses a drive train 14" having, at a far end, a prosthesis engaging thread 124, and at the opposite end, a handle 20" which facilitates turning of the drive train by the operator. The housing 12" may be C-shaped, as shown, in order to minimize the invasiveness of the surgery by better clearing anatomical structures and tissue. A dual purpose locking mechanism, best shown in FIGs. **10B** and **10C****,** is made up of a latch housing 180 which is constrained against rotation by a key 182 and slot 184 and urged part-way into the slot 184 toward the coupling end 186 of the inserter 10" by a spring 190 captured between the latch housing 180 and a shaft 192 of the drive train 14". A retainer 193 enters a slot 195 in the component 200 in order to prevent relative axial movement of the drive train 14" when the latch housing 180 is in the locked position. The spring 190 urges the latch housing 180 against a cam stop 194 when a trigger 196 is positioned in a position so as to enable the drive train 14" to be turnable within the housing 10" by the operator rotating the handle 20". The cam stop 194 is connected to a shaft 200 to which an actuator component 202 is attached, to enable a user to turn the cam stop 196 in a position to block further movement of the latch housing 180 into the recess 184. When the cam stop 196 is turned so that it does not block further entry of the latch housing 180 into the recess 184, catches or teeth 206 inside the latch housing 180 are urged into engagement with serrations 26 cut into the outer circumference of the component 212 of the drive train 14". Because the latch housing 180 is constrained against rotational movement, the engagement of the catches 206 into the serrations 210 locks the drive train against rotational movement. This enables the surgeon to pre-set and lock the position of the prosthesis 11 prior to the installation thereof. Note that orientation of the prosthesis 11 is important because the prosthesis often has pre-drilled holes 4 and thus, these must be properly positioned prior to fastening through these holes.

The "easily cleaned" objective of the invention 10, 10', 10" enables access to all surfaces that they can be cleaned (parts covering another part can be moved or removed to expose all surfaces), the reduction in number of small radius internal corners, crevices and small gaps and the absence of blind holes.

In the embodiment shown, the device 10" is disassembled for cleaning by simply urging back on the latch housing 180 against the action of the spring 190 using a knurled surface 214 designed for that purpose. The latch housing 180 is urged back until a cross pin 182 is cleared of the recess 184 so that the drive train 14" can be pivoted away from the housing 12' so as to be approximately aligned with a central shaft 216 of the drive train. In the approximately aligned position, a slide-fit connection between the forward and rearward assemblies 220, 222 of the drive train 14" are separable, thus enabling essentially the entire rearward assembly of the drive train to be removed from the housing 12" for cleaning. Note the drive train 14" is immobilized during this urging of the latch housing 180 backwards due to the angled orientation of one component of the drive train about a corner or bend in the drive train.

In an advantage, the inserter 10' is simple and easy to use, without complex and possibly confusing locks activated with the thumb.

In another advantage, it is simple to select a desired orientation of the prosthesis 11.

In another advantage, due to the drawing of the prothesis 2 against the impaction head 40, the connection between the prosthesis 11 is robust as the connection is made without any play or gaps therebetween, ensuring good support during impaction.

An objective is to provide an inserter 10, 10', 10" that is easy to disassemble and for which the disassembly is easy to learn.

Another object of the invention is to provide a dual mechanism that uses common components to lock the implant in place as well as to provide for easy disassembly for cleaning and stelilization.

Another object of the invention is to minimise the number of pieces and the risk that parts could be lost.

The object of the invention is to provide an inserter 10, 10', 10" which enables the implant to be locked in an angular orientation prior to installation of the implant.

Multiple variations and modifications are possible in the embodiments of the invention described here. Although certain illustrative embodiments of the invention have been shown and described here, a wide range of modifications, changes, and substitutions is contemplated in the foregoing disclosure. In some instances, some features of the present invention may be employed without a corresponding use of the other features. Accordingly, it is appropriate that the foregoing description be construed broadly and understood as being given by way of illustration and example only, the scope of the invention being limited only by the appended claims.

## Claims

1. An acetabular impactor (10, 10', 10") for aiding a surgeon in controlling the installation of a hip prosthesis (11), the impactor comprising:
(a) an impactor head (20);
(b) a housing (12, 12', 12") attached to the impactor head, and enclosing a drive train (14, 14', 14") having, at a far end (134), a prosthesis engaging thread (124), and at the opposite end (42'), a handle (20, 20', 20") which facilitates turning of the drive train by the operator; and
(c) a locking mechanism (44, 50, 52, 54, 56, 60, 62, 67, 68; 124, 130, 142, 146; 180, 193, 194, 195, 196, 200, 202, 206, 210, 212, 14) associated with the housing which selectively locks the drive train, and thus the prosthesis, in position, the impactor **characterised in that** the impactor housing has at least one bend permitting the housing to avoid anatomical structures or tissue during use in surgery and **in that** a link lever (42) cooperates with the prosthesis engaging thread and drive train to enable drawing of the prosthesis securely against an impaction surface (140a) of the impactor head.

2. The acetabular impactor (10, 10', 10") of claim 1, wherein the drive train (14, 14', 14") includes at least one u-joint (30') located so as to transmit torque through the bend in the housing (12, 12', 12").

3. The acetabular impactor (10, 10', 10") of claim 1, wherein the housing (12, 12', 12") is C-shaped.

4. The acetabular impactor (10) of claim 1,
wherein further the opposite end (42') of the drive train has a latch device (52, 54, 56, 60, 62; 44, 50, 180) which enables quick removal from the housing for cleaning and sterilization, and
wherein the link lever (42) is disposed in the housing (12) so as to rotate on a fulcrum (32), such that, actuation of the lever link draws the threaded tip (146a) into the housing and, when connected to a prosthesis (11), draws the prosthesis against an impaction surface (140a), wherein sufficient friction may be generated therebetween to lock the prosthesis in place.

5. The acetabular impactor (10) of claim 4, wherein the link lever (42) has a knob (20) attached to its extreme end, the knob enabling a user to orient the tip (146a).

6. The acetabular impactor (10) of any one of claims 1-5, wherein a lockable, variable length link (56) is attached between the link lever (42) and the housing (12) in order to permit a user to vary pressure that the tip can exert against the impaction head (140).

7. The acetabular impactor (10) of claim 6, wherein the variable link (56) is infinitely variable and unlockable via a latch (68) in order to permit release of pressure on the prosthesis (11).

8. The acetabular impactor (10) of claim 7, wherein the prosthesis engaging tip (146a) is connoted by way of a first U-joint (30') to a lever (32) which slides in a pivoting sleeve (34) fixed to the housing via a first pivot (36).

9. The acetabular impactor (10) of claim 1, wherein a one-way catch mechanism (67) prevents a rod (56) connected to the second lever (42) from sliding out of the housing (12) unless an unlock lever (68) is activated.

10. The acetabular impactor (10, 10', 10") of claim 1, wherein the impactor head (20) is covered by an impactor head covering (140), made of a shock-absorbing material, in order to absorb the impact stresses incurred during use of the impactor.

11. Art acetabular impactor (10') of claim 1, wherein the locking mechanism (124, 130, 142, 146) is an expandable collet (120) which a knob (20'), adjacent the handle (60), expands when turned in one direction so as to lock the collet (120) against a surface of prosthesis (11) in order to prevent the prosthesis from rotation, thus enabling the surgeon to pre-set and lock the position of the prosthesis prior to the installation thereof.

12. The impactor (10') of claim 11, wherein the collet (120) is comprised of two jaws (124) having opposite ends (125, 126) pivoting on a fulcrum (32), one end of which being adapted to engage an interior surface of a prosthesis (11), the prosthesis engaging ends being drawn away from one another when a actuator piston (146), which passes through the fulerum (130), is draw therebetween, thereby eliminating the need of threading the acetabular prosthesis (11) onto the tip (125) of the impactor as the prosthesis can simply be placed over the collet and the collet expanded so as to grip the internal threads (122) of the prosthesis.

13. The impactor (10') of claim 12, wherein the fulerum (32) is mounted in a cage (142) through which the actuator piston (146) passes, the actuator piston having a shoulder (143) bearing against a surface (142') of the cage opposite the prosthesis engaging ends (125) of the jaws (124), such that, as the actuator piston is being activated to separate the prosthesis engaging ends of the jaws, a shoulder (146e) of the piston contacting the surface compresses the jaws into the cage, thereby drawing the jaws into the impactor and, when connected to a prosthesis, thereby drawing the prosthesis against an impaction surface (140a) so as to firmly fix the prosthesis against the impaction surface.

14. the impactor (10') of claim 13, wherein the collet (120) is provided with external, three-dimensional structures (124) which engage with corresponding structures (1.22) on the prosthesis (11).

15. The impactor (10') or claim 14, wherein the three dimensional structures are threads (122).

16. The impactor (10') of claim 14, wherein the three-dimensional structures arc grooves.

17. The impactor (10') of claim 14, wherein the three-dimensional structures are divots.

18. The acetabular impactor (10') of claim 11, wherein the drive train (14') includes at least one u-joint (30') located so as to transmit torque through the bend in the housing (12').

19. The acetabular impactor (10') of claim 11 , wherein the housing (12') is C-shaped.

20. The acetabular impactor (10") of claim 1, wherein further, the locking mechanism (180, 193, 194, 195, 196, 200, 202, 206, 210, 212) is made up of a latch housing (180) which is constrainable against rotation while being urged part-way into a recess (184) toward the engagement end (186) of an impactor head (140) by a spring (190) captured between the latch housing (180) and a shaft (212) of the drive train, the spring urging the latch housing against a cam stop (194) when a trigger (196, 200) is positioned so as to selectively:
i) enable the drive train to be turnable within the housing (12'') by the operator rotating the handle (160), the cam stop (194) being connected to a shaft (200) to which an actuator component (196) is attached,
ii) enable a user to turn the cam stop (194) in a position to block further movement of the latch housing (180) into the recess (184), such that when the cam stop is turned so that it does not block further entry of the latch housing into the recess (184), catches (206) inside the latch housing are urged into engagement with serrations (210) cut into the outer circumference of a component (212) of the drive train, wherein the engagement of the catches (206) into the serrations (210) constrains the latch housing (190) against rotational movement and locks the drive train (14") against rotational movement,
the selectivity enabling the surgeon to pre-set and lock the position of the prosthesis (11) prior to the installation thereof, wherein the latch housing (180) may be unlatched from the housing so as to enable quick and invention is easily cleanable.

21. The acetabular impactor (10") of claim 1, wherein the housing (12") is C-shaped, in order to minimize the invasiveness of the surgery by better clearing anatomical structures and tissue.

## Patentansprüche

1. Hüftgelenkspfannenimpaktor (10, 10', 10") zur Unterstützung eines Chirurgen bei der Kontrolle des Einsetzens einer Hüftprothese (11), wobei der Impaktor folgendes umfasst:
(a) einen Impaktorkopf (20),
(b) ein am Impaktorkopf befestigtes Gehäuse (12, 12', 12"), das einen Antriebsstrang (14, 14', 14") umgibt, der an einem entfernten Ende (134) ein in die Prothese eingreifendes Gewinde (124) und am entgegengesetzten Ende (42') einen Handgriff (20, 20', 20") aufweist, der Drehen des Antriebsstrangs durch den Chirurgen erleichtert; und
(c) einen Arretiermechanismus (44, 50, 52, 54, 56, 60, 62, 67, 68; 124, 130, 142, 146; 180, 193, 194, 195, 196, 200, 202, 206, 210, 212, 14) in Verbindung mit dem Gehäuse, der den Antriebsstrang und somit die Prothese selektiv in ihrer Position arretiert, wobei der Impaktor **dadurch gekennzeichnet ist, dass** das Impaktorgehäuse mindestens eine Biegung aufweist, die es dem Gehäuse ermöglicht, anatomische Strukturen oder Gewebe im Gebrauch bei einer Operation zu umgehen, und dass eine Hebelverbindung (42) mit dem in die Prothese eingreifenden Gewinde und dem Antriebsstrang zusammenarbeitet, damit die Prothese fest gegen eine Schlagfläche (140a) des Impaktorkopfes gezogen werden kann.

2. Hüftgelenkspfannenimpaktor (10, 10', 10") nach Anspruch 1, worin der Antriebsstrang (14, 14', 14") mindestens ein U-Gelenk (30') aufweist, das so angeordnet ist, dass es ein Drehmoment durch eine Biegung im Gehäuse (12, 12', 12") überträgt.

3. Hüftgelenkspfannenimpaktor (10, 10', 10") nach Anspruch 1, worin das Gehäuse (12, 12', 12") C-förmig ist.

4. Hüftgelenkspfannenimpaktor (10) nach Anspruch 1, worin das entgegengesetzte Ende (42') des Antriebsstrangs ferner eine Riegelvorrichtung (52, 54, 56, 60, 62; 44, 50; 180) aufweist, die schnelles Entfernen vom Gehäuse zur Reinigung und Sterilisation ermöglicht, und worin eine Hebelverbindung (42) im Gehäuse (12) angeordnet ist, um auf einem Drehpunkt (32) zu drehen, so dass die Betätigung der Hebelverbindung die Gewindespitze (146a) in das Gehäuse zieht und bei Verbindung mit einer Prothese (11) die Prothese gegen eine Schlagfläche (140a) zieht, worin dazwischen ausreichend Reibung erzeugt werden kann, um die Prothese zu arretieren.

5. Hüftgelenkspfannenimpaktor (10) nach Anspruch 4, worin die Hebelverbindung (42) einen am extremen Ende befestigten Knopf (20) aufweist, der es einem Anwender ermöglicht, die Spitze (146a) zu orientieren.

6. Hüftgelenkspfannenimpaktor (10) nach einem der Ansprüche 1-5, worin eine arretierbare Verbindung (56) variabler Länge zwischen der Hebelverbindung (42) und dem Gehäuse (12) befestigt ist, damit ein Anwender den Druck variieren kann, den die Spitze gegen den Impaktorkopf (140) aufbringen kann.

7. Hüftgelenkspfannenimpaktor (10) nach Anspruch 6, worin die variable Verbindung (56) unendlich variabel ist und über einen Riegel (68) gelöst werden kann, um den Druck auf die Prothese (11) zu entlasten.

8. Hüftgelenkspfannenimpaktor (10) nach Anspruch 7, worin die in die Prothese eingreifende Spitze (146a) über ein erstes U-Gelenk (30') mit einem Hebel (32) verbunden ist, der in einer über einen ersten Drehzapfen (36) am Gehäuse befestigten Schwenkhülse (34) gleitet.

9. Hüftgelenkspfannenimpaktor (10) nach Anspruch 1, worin ein Einwege-Riegelmechanismus (67) verhindert, dass eine mit dem zweiten Hebel (42) verbundene Stange (56) aus dem Gehäuse (12) gleitet, wenn kein Lösehebel (68) aktiviert wird.

10. Hüftgelenkspfannenimpaktor (10, 10', 10") nach Anspruch 1, worin der Impaktorkopf (20) von einer Impaktorkopfabdeckung (140) aus einem stoßdämpfenden Material abgedeckt ist, um die im Gebrauch des Impaktors entstehenden Stoßbeanspruchungen aufzunehmen.

11. Hüftgelenkspfannenimpaktor (10') nach Anspruch 1, worin der Arretiermechanismus (124, 130, 142, 146) eine expandierbare Klemmhülse (120) ist, die ein Knopf (20') neben dem Handgriff (60) expandiert, wenn er in eine Richtung gedreht wird, um die Klemmhülse (120) gegen eine Fläche der Prothese (11) zu arretieren um zu verhindern, dass sich die Prothese dreht, so dass der Chirurg die Position der Prothese vor dem Einsetzen festlegen und arretieren kann.

12. Impaktor (10') nach Anspruch 11, worin die Klemmhülse (120) aus zwei Backen (124) besteht, deren entgegengesetzten Enden (125, 126) auf einem Drehpunkt (32) drehen, wobei ein Ende in eine Innenfläche einer Prothese (11) eingreifen kann, die in die Prothese eingreifenden Enden voneinander weggezogen werden, wenn ein Betätigungskolben (146), der durch den Drehpunkt (130) verläuft, dazwischen gezogen wird, wodurch die Notwendigkeit des Aufschraubens der Hüftgelenkspfannenprothese (11) auf die Spitze (125) des Impaktors entfällt, da die Prothese einfach über die Klemmhülse gesetzt und die Klemmhülse expandiert werden kann, um die Innengewinde (122) der Prothese zu ergreifen.

13. Impaktor (10') nach Anspruch 12, worin der Drehpunkt (32) in einem Käfig (142) befestigt ist, durch den der Betätigungskolben (146) verläuft, wobei der Betätigungskolben eine Schulter (143) aufweist, die an einer Fläche (142') des Käfigs gegenüber den in die Prothese eingreifenden Enden (125) der Backen (124) anliegt, so dass bei Aktivierung des Betätigungskolbens zum Trennen der in die Prothese eingreifenden Enden der Backen eine Schulter (146e) des Kolbens, die mit der Fläche in Berührung ist, die Backen in den Käfig drückt, wodurch die Backen in den Impaktor gezogen werden und bei Verbindung mit einer Prothese die Prothese gegen eine Schlagfläche (140a) gezogen wird, um die Prothese sicher an der Schlagfläche zu fixieren.

14. Impaktor (10') nach Anspruch 13, worin die Klemmhülse (120) mit externen dreidimensionalen Strukturen (124) versehen ist, die in entsprechende Strukturen (122) auf der Prothese (11) eingreifen.

15. Impaktor (10') nach Anspruch 14, worin die dreidimensionalen Strukturen Gewinde (122) sind.

16. Impaktor (10') nach Anspruch 14, worin die dreidimensionalen Strukturen Rillen sind.

17. Impaktor (10') nach Anspruch 14, worin die dreidimensionalen Strukturen Flächenstücke sind.

18. Hüftgelenkspfannenimpaktor (10') nach Anspruch 11, worin der Antriebsstrang (14') mindestens ein U-Gelenk (30') aufweist, das so angeordnet ist, dass es ein Drehmoment an einer Biegung im Gehäuse (12') überträgt.

19. Hüftgelenkspfannenimpaktor (10') nach Anspruch 11, worin das Gehäuse (12') C-förmig ist.

20. Hüftgelenkspfannenimpaktor (10') nach Anspruch 1, worin der Arretiermechanismus (180, 193, 194, 195, 196, 200, 202, 206, 210, 212) ferner aus einem Riegelgehäuse (180) besteht, das gegen Drehung festgehalten werden kann, während es von einer Feder (190), die zwischen dem Riegelgehäuse (180) und einem Schaft (212) des Antriebsstrangs festgehalten wird, teilweise in eine Vertiefung (184) zum Eingriffsende (186) des Impaktorkopfes (140) gedrückt wird, wobei die Feder das Riegelgehäuse gegen einen Nockenanschlag (194) drückt, wenn ein Auslöser (196, 200) so positioniert ist, dass wahlweise
i) der Antriebsstrang im Gehäuse (12") gedreht werden kann, indem der Chirurg den Handgriff (160) dreht, wobei der Nockenanschlag (194) mit einem Schaft (200) verbunden ist, an dem eine Betätigungskomponente (196) befestigt ist,
ii) ein Anwender den Nockenanschlag (194) in eine Position drehen kann, um die weitere Bewegung des Riegelgehäuses (180) in die Vertiefung (184) zu verhindern, dass er beim Drehen des Nockenanschlags den weiteren Eintritt des Riegelgehäuses in die Vertiefung (184) nicht blockiert, Fallen (206) werden im Riegelgehäuse in Eingriff mit Verzahnungen (210) im Außenumfang einer Komponente (212) des Antriebsstrangs gedrückt, worin der Eingriff der Fallen (206) in die Verzahnungen (210) das Riegelgehäuse (190) gegen Drehbewegung festhält und den Antriebsstrang (14") gegen Drehbewegung arretiert,
wobei es diese Selektivität dem Chirurgen ermöglicht, die Position der Prothese (11) vor dem Einsetzen festzulegen und zu arretieren, worin das Riegelgehäuse (180) vom Gehäuse gelöst werden kann, damit die Erfindung schnell und leicht gereinigt werden kann.

21. Hüftgelenkspfannenimpaktor (10') nach Anspruch 1, worin das Gehäuse (12") C-förmig ist, um den invasiven Charakter der Operation zu verringern, indem anatomische Strukturen und Gewebe besser zur Seite geräumt werden können.

## Revendications

1. Impacteur acétabulaire (10, 10', 10") permettant d'aider un chirurgien à contrôler l'installation d'une prothèse de la hanche (11), l'impacteur comprenant:
(a) une tête d'impacteur (20) ;
(b) un logement (12, 12', 12") attaché à la tête d'impacteur et renfermant un système d'entraînement (14, 14', 14") muni, à une extrémité distale (134), d'un filet (124) pour engager la prothèse, et à l'autre extrémité (42'), d'une poignée (20, 20', 20") qui facilite la rotation du système d'entraînement par l'opérateur ; et
(c) un mécanisme de verrouillage (44, 50, 52, 54, 56, 60, 62, 67, 68 ; 124, 130, 142, 146 ; 180, 193, 194, 195, 196, 200, 202, 206, 210, 212, 214) associé au logement, qui verrouille de manière sélective le système d'entraînement, et donc la prothèse, en place, l'impacteur étant **caractérisé en ce que** le logement d'impacteur a au moins une courbure permettant au logement d'éviter des structures anatomiques ou des tissus au cours de l'utilisation pendant une opération chirurgicale et **en ce qu'**une liaison de levier (42) coopère avec le filet pour engager la prothèse et avec le système d'entraînement pour permettre de tirer la prothèse de manière sûre contre une surface d'impact (104a) de la tête d'impacteur.

2. Impacteur acétabulaire (10, 10', 10") selon la revendication 1, dans lequel le système d'entraînement (14, 14', 14") comporte au moins un joint en U (30') situé de manière à transmettre le couple à travers la courbure dans le logement (12, 12', 12").

3. Impacteur acétabulaire (10, 10', 10") selon la revendication 1, dans lequel le logement (12, 12', 12") est en forme de C.

4. Impacteur acétabulaire (10) selon la revendication 1, dans lequel, en outre, l'extrémité opposée (42') du système d'entraînement a un dispositif d'encliquetage (52, 54, 56, 60, 62 ; 44, 50 ; 180) qui permet un retrait rapide du logement en vue du nettoyage et de la stérilisation, et dans lequel la liaison de levier (42) est disposée dans le logement (12) de manière à tourner sur un point d'appui (32), de telle sorte que l'actionnement de la liaison de levier tire la pointe filetée (146a) dans le logement, et lorsqu'elle est connectée à une prothèse (11), tire la prothèse contre une surface d'impact (140a), un frottement suffisant pouvant être produit entre eux pour verrouiller la prothèse en place.

5. Impacteur acétabulaire (10) selon la revendication 4, dans lequel la liaison de levier (42) a un bouton (20) attaché tout au bout de son extrémité, le bouton permettant à un utilisateur d'orienter la pointe (146a).

6. Impacteur acétabulaire (10) selon l'une quelconque des revendications 1 à 5, dans lequel une liaison verrouillable de longueur variable (56) est attachée entre la liaison de levier (42) et le logement (12) afin de permettre à un utilisateur de faire varier la pression que la pointe peut exercer contre la tête d'impacteur (140).

7. Impacteur acétabulaire (10) selon la revendication 6, dans lequel la liaison variable (56) est infiniment variable et peut être déverrouillée par le biais d'un cliquet (68) afin de permettre de relâcher la pression appliquée à la prothèse (11).

8. Impacteur acétabulaire (10) selon la revendication 7, dans lequel la pointe (146a) engageant la prothèse est connectée par le biais d'un premier joint en U (30') à un levier (32) qui glisse dans un manchon pivotant (34) fixé au logement par le biais d'un premier pivot (36).

9. Impacteur acétabulaire (10) selon la revendication 1, dans lequel un mécanisme d'encliquetage unidirectionnel (67) empêche une tige (56) connectée au deuxième levier (42) de glisser hors du logement (12) tant qu'un levier de déverrouillage (68) n'est pas activé.

10. Impacteur acétabulaire (10, 10', 10") selon la revendication 1, dans lequel la tête d'impacteur (20) est recouverte par un recouvrement de tête d'impacteur (140) fabriqué en matériau absorbant les chocs, afin d'absorber les contraintes d'impact occasionnées au cours de l'utilisation de l'impacteur.

11. Impacteur acétabulaire (10') selon la revendication 1, dans lequel le mécanisme de verrouillage (124, 130, 142, 146) est un collet écartable (120) qu'un bouton (20'), adjacent à la poignée (60), écarte lorsqu'il est tourné dans un sens de manière à verrouiller le collet (120) contre une surface d'une prothèse (11) afin d'empêcher la prothèse de tourner, en permettant ainsi au chirurgien de préfixer et de verrouiller la position de la prothèse avant son installation.

12. Impacteur (10') selon la revendication 11, dans lequel le collet (120) se compose de deux mâchoires (124) ayant des extrémités opposées (125, 126) pivotant sur un point d'appui (32), dont une extrémité est adaptée pour s'engager avec une surface intérieure d'une prothèse (11), les extrémités engageant la prothèse étant écartées l'une de l'autre lorsqu'un piston d'actionnement (146), qui passe à travers le point d'appui (130), est tiré entre elles, en éliminant ainsi la nécessité d'un filetage de la prothèse acétabulaire (11) sur la pointe (125) de l'impacteur, car la prothèse peut être simplement placée par-dessus le collet et le collet écarté de manière à saisir les filets internes (122) de la prothèse.

13. Impacteur (10') selon la revendication 12, dans lequel le point d'appui (32) est monté dans une cage (142) à travers laquelle passe le piston d'actionnement (146), le piston d'actionnement ayant un épaulement (143) pressant contre une surface (142') de la cage en face des extrémités (125) des mâchoires (124) engageant la prothèse, de telle sorte que lorsque le piston d'actionnement est activé pour séparer les extrémités des mâchoires engageant la prothèse, un épaulement (146e) du piston en contact avec la surface comprime les mâchoires dans la cage, en tirant ainsi les mâchoires dans l'impacteur, et, lorsqu'elles sont connectées à une prothèse, en tirant ainsi la prothèse à l'encontre d'une surface d'impact (140a) de manière à fixer fermement la prothèse contre la surface d'impact.

14. Impacteur (10') selon la revendication 13, dans lequel le collet (120) est pourvu de structures externes tridimensionnelles (124) qui s'engagent avec des structures correspondantes (122) sur la prothèse (11).

15. Impacteur (10') selon la revendication 14, dans lequel les structures tridimensionnelles sont des filets (122).

16. Impacteur (10') selon la revendication 14, dans lequel les structures tridimensionnelles sont des gorges.

17. Impacteur (10') selon la revendication 14, dans lequel les structures tridimensionnelles sont des creux.

18. Impacteur acétabulaire (10') selon la revendication 11, dans lequel le système d'entraînement (14') comporte au moins un joint en U (30') situé de manière à permettre de transmettre le couple par une courbe dans le logement (12').

19. Impacteur acétabulaire (10') selon la revendication 11, dans lequel le logement (12') est en forme de C.

20. Impacteur acétabulaire (10") selon la revendication 1, dans lequel, en outre, le mécanisme de verrouillage (180, 193, 194, 195, 196, 200, 202, 206, 210, 212) est constitué d'un logement d'encliquetage (180) qui peut être bloqué en rotation tout en étant poussé en partie dans un retrait (184) vers l'extrémité d'engagement (186) d'une tête d'impacteur (140) par un ressort (190) reçu entre le logement d'encliquetage (180) et une tige (212) du système d'entraînement, le ressort poussant le logement d'encliquetage contre une butée à came (194) lorsqu'une gâchette (196, 200) est positionnée de manière à, sélectivement :
i) permettre au système d'entraînement de tourner dans le logement (12") sous l'effet de la rotation de la poignée (160) par l'opérateur, la butée à came (194) étant connectée à une tige (200) à laquelle un composant d'actionneur (196) est attaché,
ii) permettre à un utilisateur de faire tourner la butée à came (194) dans une position bloquant davantage le mouvement du logement d'encliquetage (180) dans le retrait (184), de telle sorte que lorsque la butée à came est tournée de manière à ce qu'elle ne bloque plus davantage l'entrée du logement d'encliquetage dans le retrait (184), des cliquets (206) à l'intérieur du logement d'encliquetage soient poussés en engagement avec des indentations (210) découpées dans la circonférence extérieure d'un composant (212) du système d'entraînement, l'engagement des cliquets (206) dans les indentations (210) bloquant le logement d'encliquetage (190) contre tout mouvement de rotation et verrouillant le système d'entraînement (14") contre tout mouvement de rotation,
la sélectivité permettant au chirurgien de préfixer et de verrouiller la position de la prothèse (11) avant son installation, le logement d'encliquetage (180) pouvant être déverrouillé du logement de manière à permettre un retrait rapide et un nettoyage aisé.

21. Impacteur acétabulaire (10") selon la revendication 1, dans lequel le logement (12") est en forme de C, afin de minimiser le caractère invasif de la chirurgie en permettant de passer plus facilement à travers les structures anatomiques et les tissus.
